# EUROPÄISCHE PATENTSCHRIFT

(11) **EP 0 911 340 B1**
(45) Veröffentlichungstag und Bekanntmachung des Hinweises auf die Patenterteilung: **06.07.2005**
(21) Anmeldenummer: 98119591.0
(22) Anmeldetag: 16.10.1998
(51) Int. Cl.: C07K 5/078, C07D 217/26, A61K 38/05, A61K 31/47

(54) **Substituierte Isochinolin-3-Carbonsäureamide, ihre Herstellung und ihre Verwendung als Arzneimittel**
Substituted Isoquinolin-3-Carboxyamides, their preparation and medical use
Amides de l'acide 3-isoquinoléique substitués, leur préparation et leur utilisation médicale

(30) Priorität: 20.10.1997 DE 19746287
(43) Veröffentlichungstag der Anmeldung: 28.04.1999
(60) Teilanmeldung: 05004047.6
(73) Patentinhaber: FibroGen, Inc., South San Francisco, CA 94080 (US)
(72) Erfinder: Weidmann, Klaus, Dr., 61476 Kronberg (DE); Baringhaus, Karl-Heinz, Dr., 61200Wölfersheim (DE); Tschank, Georg, Dr., 55270 Essenheim (DE); Werner, Ulrich, Dr., 56357 Miehlen (DE)
(74) Vertreter: UEXKÜLL & STOLBERG

(56) Entgegenhaltungen:
- EP-A- 0 548 883
- EP-A- 0 650 961
- EP-A- 0 661 269
- EP-A- 0 765 871
- FRANKLIN T J ET AL: "APPROACHES TO THE DESIGN OF ANTI-FIBROTIC DRUGS" BIOCHEMICAL SOCIETY TRANSACTIONS, Bd. 19, 1991, Seiten 812-815, XP002091212

## Beschreibung

Die Erfindung betrifft substituierte Isochinolin-3-carbonsäureamide, ihre Herstellung und ihre Verwendung als Inhibitoren der Prolyl-4-hydroxylase und ihre Verwendung als Arzneimittel zur Behandlung von fibrotischen Erkrankungen.

Verbindungen, die das Enzym Prolylhydroxylase inhibieren, bewirken eine sehr selektive Hemmung der Kollagenbiosynthese durch Beeinflussung der kollagenspezifischen Hydroxylierungsreaktionen. In deren Verlauf wird proteingebundenes Prolin oder Lysin durch die Enzyme Prolyl- bzw. Lysylhydroxylase hydroxyliert. Wird diese Reaktion durch Inhibitoren unterbunden, so entsteht ein nicht funktionsfähiges, unterhydroxyliertes Kollagenmolekül, das von den Zellen nur in geringer Menge in den extrazellulären Raum abgegeben werden kann. Das unterhydroxylierte Kollagen kann außerdem nicht in die Kollagenmatrix eingebaut werden und wird sehr leicht proteolytisch abgebaut. Als Folge dieser Effekte verringert sich insgesamt die Menge des extrazellulär abgelagerten Kollagens.

Inhibitoren der Prolylhydroxylase sind deshalb geeignete Substanzen in der Therapie von Erkrankungen, in denen die Ablagerung von Kollagenen maßgeblich zum Krankheitsbild beiträgt. Hierzu gehören u. a. Fibrosen der Lunge, Leber und Haut (Skleroderma und Vernarbungen nach Verbrennungen, Verletzungen und chirurgischen Eingriffen) und sowie die Atherosklerose.

Es ist bekannt, daß das Enzym Prolylhydroxylase durch Pyridin-2,4- und -2,5-dicarbonsäure effektiv gehemmt wird (K. Majamaa et al., Eur. J. Biochem. 138 (1984) 239-245). Diese Verbindungen sind in der Zellkultur allerdings nur in sehr hohen Konzentrationen als Hemmstoffe wirksam (Tschank, G. et al., Biochem. J. 238 (1987) 625 bis 633).

Auch Prodrugs der Pyridin-2,4(5)-dicarboxylate sind bekannt. Diese sind in den EP-A-0 590 520 und EP-A-0 562 512 beschrieben.

N-Oxalylglycine als Inhibitoren der Prolyl-4-hydroxylase sind aus J. Med. Chem. 1992, 35, 2652 bis 2658 (Cunliffe et al.), und EP-A-0 457 163 (Baader et al.) bekannt.

3-Hydroxypyridin-2-carbonsäure-N-(carboxymethyl)amid ist aus G. Yolles et al. in: Bull. Soc. Chim. Fr. 1965, 8, 2252 bis 2259 bekannt.

N-((4-Hydroxyisochinolin-3-yl)carbonyl)glycin und N-((7-Brom-4-hydroxyisochinolin-3-yl)carbonyl)glycin sind aus Biochem. Soc. Trans. 1991, 19, 812 bis 815 (Franklin et al.) bekannt, wobei im Falle von N-((4-Hydroxyisochinolin-3-yl)carbonyl)glycin die in vivo Aktivität auf die Kollagenbiosynthese schwach ist. Auch Hydroxychinolincarbonsäureglycylamide sind hier genannt. In T.J. Franklin in Therapeutic Approaches to Organ Fibrosis , Int. J. Biochem. Cell. Biol., 1997, Vol. 29, No. 1, 79 - 89 wird im Falle von N-((7-Brom-4-hydroxyisochinolin-3-yl)carbonyl)glycin neben der in vivo Hemmung der Kollagenbiosysthese von einer toxischen Wirkung auf die Leber (Steatose) an Ratten berichtet.

In der EP-A-0 661 269 werden substituierte heterocyclische Carbonsäureamide und ihre Verwendung als Inhibitoren der Prolyl-4-hydroxylase und als Hemmstoffe der Kollagenbiosynthese beschrieben.

Es bestand die Aufgabe, nach stärker wirksamen Inhibitoren der Prolylhydroxylase zu suchen. Weiterhin bestand die Aufgabe, nach wirksamen Inhibitoren der Prolylhydroxylase zu suchen, die keine Steatose bewirken.

Es wurde nun gefunden, daß neue Isochinolin-3-carbonsäureamide überraschend starke Prolyl-4-hydroxylase-Inhibitoren sind, die nicht Steatose bewirken.

Die erfindungsgemäßen Verbindungen entsprechen der Formel I 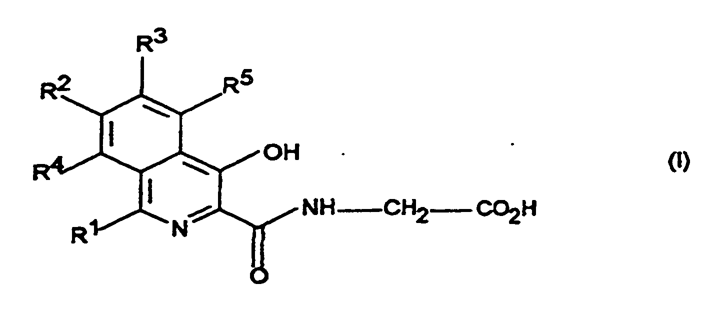 in welcher
- R¹: Chlor,
- R²: Wasserstoff, (C₁-C₈)-Alkyl, (C₁-C₈)-Alkoxy, Chlor, Trifluormethyl, Hydroxy, oder
Benzyloxy, das gegebenenfalls mit Substituenten aus der Reihe (C₁-C₅)-Alkyl, (C₁-C₅)-Alkoxy substituiert ist, oder
Fluoralkoxy der Formel

O-[CH₂]ₓ-C_{f}H_{(2f-1-g)}F_{g}

mit
x = O oder 1,
f = 1 - 5 und
g = 1 bis (2f+1),
- R³: Wasserstoff, (C₁-C₈)-Alkyl, (C₁-C₈)-Alkoxy, Fluor, Chlor, Cyano, Trifluormethyl, Hydroxy, oder
Benzyloxy, das gegebenenfalls mit Substituenten aus der Reihe (C₁-C₅)-Alkyl, (C₁-C₅)-Alkoxy substituiert ist, oder
Fluoralkoxy der Formel

O-[CH₂]ₓ-C_{f}H_{(2f+1-g)}F_{g}

worin x, f und g wie oben definiert sind,
- R⁴ und R⁵: Wasserstoff, (C₁-C₅)-Alkyl, Fluor, Chlor, Brom, Trifluormethyl, Cyano, (C₁-C₅)-Alkoxy, oder
Fluoralkoxy der Formel

O-[CH₂]ₓ-C_{f}H_{(2f+1-g)}F_{g}

worin x, f und g wie oben definiert sind,
einschließlich der physiologisch wirksamen Salze.

Bevorzugt sind Verbindungen der Formel I, in welcher
- R¹: Chlor,
- R²: Wasserstoff, (C₁-C₈)-Alkyl, (C₁-C₈)-Alkoxy, Chlor, Trifluormethyl, Hydroxy,
Benzyloxy, oder
Fluoralkoxy der Formel

O-[CH₂]ₓ-C_{f}H_{(2f+1-g)}F_{g}

mit
x = O oder 1,
f = 1 - 5 und
g = 1 bis (2f+ 1),
- R³: Wasserstoff, (C₁-C₈)-Alkyl, (C₁-C₈)-Alkoxy, Fluor, Chlor, Cyano, Trifluormethyl, Hydroxy, Benzyloxy, oder
Fluoralkoxy der Formel

O-[CH₂]ₓ-C_{f}H_{(2f+1-g)}F_{g}

worin x, f und g wie vorstehend definiert sind,
- R⁴: Wasserstoff, (C₁-C₈)-Alkoxy, Fluor, Chlor, Trifluormethyl, Cyano, oder Fluoralkoxy der Formel

O-[CH₂]ₓ-C_{f}H_{(2f+1-g)}F_{g}

worin x, f und g wie vorstehend definiert sind, und
- R⁵: Wasserstoff sind.

Ferner sind bevorzugt Verbindungen der Formel I, in welcher
- R¹: Chlor,
- R²: Wasserstoff, (C₁-C₅)-Alkoxy, Chlor, Benzyloxy, Fluoralkoxy mit x = O, f=1,
- R³: Wasserstoff, (C₁-C₅)-Alkoxy, Chlor, Benzyloxy,
- R⁴: Wasserstoff, Chlor, Methoxy,
- R⁵: Wasserstoff sind
und solche, in denen
- R¹: Chlor,
- R²: Wasserstoff, (C₁-C₈)-Alkoxy, Chlor, Hydroxy, Benzyloxy,
- R³: Wasserstoff, (C₁-C₈)-Alkoxy, Fluor, Chlor, Hydroxy, Benzyloxy,
- R⁴: Wasserstoff, Chlor,
- R⁵: Wasserstoff sind.

Besonders bevorzugt sind Verbindungen der Formel I, in welcher
- R¹: Chlor,
- R²: (C₁-C₈)-Alkoxy, Chlor, Benzyloxy, und
- R³, R⁴ und R⁵: Wasserstoff,
oder in welcher
- R¹: Chlor,
- R²: Wasserstoff oder Chlor,
- R³: (C₁-C₈)-Alkoxy, Chlor, Benzyloxy, und
- R⁴ und R⁵: Wasserstoff,
oder in welcher
- R¹: Chlor,
- R² und R³: Wasserstoff oder Chlor,
- R⁴: (C₁-C₆)-Alkoxy, Chlor, Benzyloxy, und
- R⁵: Wasserstoff.

Ferner sind besonders bevorzugt Verbindungen der Formel I, in welcher
- R¹: Chlor,
- R²: (C₁-C₆)-Alkoxy oder Benzyloxy,
- R³, R⁴ und R⁵: Wasserstoff,
oder in welcher,
- R¹: Chlor,
- R²: Wasserstoff,
- R³: (C₁-C₆)-Alkoxy oder Benzyloxy, und
- R⁴ und R⁵: Wasserstoff.

Insbesondere bevorzugt sind Verbindungen der Formel I, in welcher
- R¹: Chlor,
- R²: (C₁-C₆)-Alkoxy, und
- R³, R⁴ und R⁵: Wasserstoff,
oder in welcher,
- R¹: Chlor,
- R²: Wasserstoff,
- R³: (C₁-C₆)-Alkoxy, und
- R⁴ und R⁵: Wasserstoff.

Ganz besonders seien genannt:
N-((1-Chlor-4-hydroxyisochinolin-3-yl)carbonyl)glycin,
N-((1-Chlor-4-hydroxy-7-((2-propyl)oxy)isochinolin-3-yl)carbonyl)glycin

Des weiteren bestand die Aufgabe, nach wirksamen Inhibitoren der Prolylhydroxylase zu suchen, die leberselektiv sind.

Es wurde nun gefunden, daß neue Isochinolin-3-carbonsäure-N-(2-hydroxyethyl)amide der Formel Ia, Alkohol-Prodrugs der entsprechenden Verbindungen der Formel I sind.

Die Prodrug-Verbindungen der Formel la werden im lebenden Organismus (in vivo) und im isolierten Organ (perfundierte Leber, in vitro) zu Verbindungen der Formel I oxidiert. Die Umwandlung der Verbindungen der Formel 1 findet bevorzugt in der Leber statt, wodurch eine leberselektive Hemmung der Prolyl-4-hydroxylase und der Kollagenbiosynthese erzielt wird.

Nach der Applikation der Verbindungen der Formel Ia bewirken sie die in vivo und in vitro zu beobachtende Hemmung der Prolyl-4-hydroxylase, wobei sich die Verbindungen der Formel I bilden. Diese Verbindungen inhibieren die Prolyl-4-hydroxylase und führen deshalb zu einer Hemmung der Kollagenbiosynthese.

Die Verbindungen entsprechen der Formel Ia 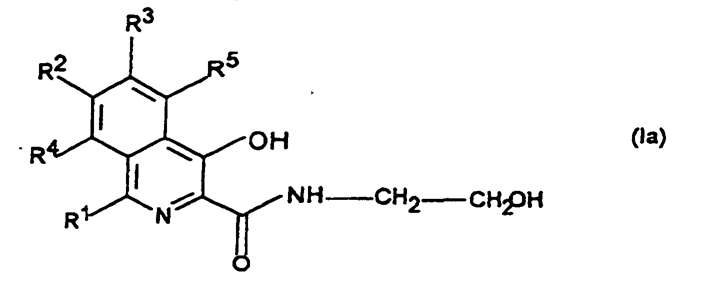 in welcher
- R¹: Chlor,
- R²: Wasserstoff, (C₁-C₈)-Alkyl, (C₁-C₈)-Alkoxy, Chlor, Trifluormethyl, Hydroxy, oder
Benzyloxy, das gegebenenfalls mit Substituenten aus der Reihe (C₁-C₅)-Alkyl, (C₁-C₅)-Alkoxy substituiert ist, oder
Fluoralkoxy der Formel

O-[CH₂]ₓ-C_{f}H_{(2f+1-g)}F_{g}

mit
x = 0 oder 1,
f = 1 - 5 und
g = 1 bis (2f + 1),
- R³: Wasserstoff, (C₁-C₈)-Alkyl, (C₁-C₈)-Alkoxy, Fluor, Chlor, Cyano, Trifluormethyl, Hydroxy, oder
Benzyloxy, das gegebenenfalls mit Substituenten aus der Reihe (C₁-C₅)-Alkyl, (C₁-C₅)-Alkoxy substituiert ist, oder
Fluoralkoxy der Formel

O-[CH₂]ₓ-C_{f}H_{(2f+1-g)}F_{g}

worin x, f und g wie oben definiert sind,
- R⁴ und R⁵: Wasserstoff, (C₁-C₅)-Alkyl, Fluor, Chlor, Brom, Trifluormethyl, Cyano, (C₁-C₅)-Alkoxy, oder
Fluoralkoxy der Formel

O-[CH₂]ₓ-C_{f}H_{(2f+1-g)}F_{g}

worin x, f und g wie oben definiert sind,
einschließlich der physiologisch wirksamen Salze.

Bevorzugt sind Verbindungen der Formel Ia, in welcher
- R¹: Chlor,
- R² und R³: Wasserstoff oder (C₁-C₄)-Alkoxy und
- R⁴ und R⁵: Wasserstoff bedeuten.

Insbesondere bevorzugt sind Verbindungen der Formel Ia, in welcher
- R¹: Chlor,
- R²: (C₁-C₆)-Alkoxy und
- R³, R⁴ und R⁶: Wasserstoff bedeuten,
oder in welcher
- R¹: Chlor,
- R²: Wasserstoff,
- R³: (C₁-C₆)-Alkoxy und
- R⁴ und R⁵: Wasserstoff bedeuten.

Ganz besonders sei genannt:
1-Chlor-4-hydroxy-7-((2-propyl)oxy)isochinolin-3-carbonsäure-N-(2-hydroxyethyl)amid.

Die Erfindung umfaßt weiterhin Salze der Verbindungen der allgemeinen Formeln I.

Die Salzbildung mit basischen Reagenzien kann ein- oder zweifach an den aciden Gruppen der Verbindungen der Formeln I, d.h. an den Resten R¹, R², R³, R⁴ und R⁵ und/oder an der aciden phenolischen OH-Gruppe, insbesondere an der phenolischen OH-Gruppe erfolgen.

Zur Anwendung kommende Reagenzien sind beispielsweise Alkoholate, Hydroxide, Carbonate, Hydrogencarbonate, Hydrogenphosphate und/oder Metallorganyle der Alkali- und Erdalkalielemente, der Elemente der 3. und 4. Hauptgruppe des Periodensystems und der Elemente der Übergangsmetalle,
Amine, gegebenenfalls 1- bis 3-fach substituiert mit (C₁-C₈)-Hydroxyalkyl, (C₁-C₄)-Alkoxy-(C₁-C₈)-alkyl, Phenyl, Benzyl oder (C₁-C₈)-Alkyl, welches 1- bis 3-fach substituiert sein kann mit Hydroxy oder (C₁-C₄)-Alkoxy, beispielsweise Tromethan (Tris-Puffer), 2-Aminoethanol, 3-Aminopropanol, Hydroxylamin, Dimethylhydroxylamin, 2-Methoxy-ethylamin, 3-Ethoxypropylamin, und
basische Aminosäuren und -derivate, wie Aminosäureester, Histidin, Arginin und Lysin und deren Derivate, sowie
Arzneimittel, die eine basische Gruppe enthalten, wie beispielsweise Amilorid, Verapamil und Betablocker.

Die Erfindung umfaßt weiterhin Prodrugs zu den Verbindungen der Formel (I), die eine Hemmung der Kollagenbiosynthese in vivo durch Freisetzung von Verbindungen der Formel I oder deren Salzen bewirken.

Schließlich umfaßt die Erfindung auch Prodrugs, die in vivo durch Freisetzung von Verbindungen der Formel I oder deren Salzen eine inhibitorische Wirkung auf die Prolyl-4-hydroxylase bewirken.

Prodrug-Gruppierungen sind chemische Gruppen, die in vivo
zur Carboxylatgruppe der Verbindungen der Formel I umgewandelt werden und/oder
vom Amid-N-Atom abgespalten werden können und/oder
zu einem Chinolinring umgewandelt werden können.

Die in Betracht kommenden Prodrug-Gruppen sind dem Fachmann bekannt.

Insbesondere sind folgende Prodrug-Gruppierungen genannt:
für die Carboxylatgruppe Ester-, Amid-, Hydroxymethyl- und Aldehydgruppen und deren Abkömmlinge, für das Chinolin-N-Atom N-Oxide und N-Alkylderivate.

Die Erfindung betrifft die Verwendung von Verbindungen der Formel I sowie die physiologisch verträglichen Salze zur Herstellung eines Arzneimittels zur Inhibierung der Kollagenbiosynthese.

Die Erfindung betrifft die Verwendung von Verbindungen der Formel I sowie die physiologisch verträglichen Salze zur Herstellung eines Arzneimittels zur Hemmung der Prolyl-4-hydroxylase.

Weiterhin betrifft die Erfindung die Verwendung von Verbindungen der Formel I sowie die physiologisch verträglichen Salze zur Herstellung eines Arzneimittels gegen fibrotische Erkrankungen.

Weiterhin betrifft die Erfindung die Verwendung von Verbindungen der Formel sowie die physiologisch verträglichen Salze zur Herstellung eines Arzneimittels gegen fibrotische Erkrankungen der Leber, der Niere, der Lunge und der Haut.

Schließlich betrifft die Erfindung die Verbindungen der Formel I zur Verwendung als Arzneimittel.
Insbesondere betrifft die Erfindung die Verbindungen der Formel I zur Anwendung als Fibrosuppressiva.

Ferner betrifft die Erfindung die Verwendung von Verbindungen der Formel Ia sowie die physiologisch verträglichen Salze zur Herstellung eines Arzneimittels gegen fibrotische Erkrankungen der Leber.

Verbindungen der Formeln I mit 4-Mercapto sind ebenfalls wirksame Inhibitoren der Prolyl-4-hydroxylase. Die entsprechenden 3-Mercapto-pyridin-2-carbonsäureamide sind aus EP-A 0 661 269 (HOE 93/F 437K) bekannt.

Weiterhin betrifft die Erfindung ein Verfahren zur Herstellung von Verbindungen der allgemeinen Formel I.

Die Herstellung der Verbindungen der Formel I, erfolgt, indem
1.i) Chinolin-2-carbonsäuren der Formel II mit den Aminoestern der Formel III zu den Amidestern der Formel IV umgesetzt werden, und
1.ii) die Verbindungen der Formeln I aus ihren Estern der Formeln IV freigesetzt werden. 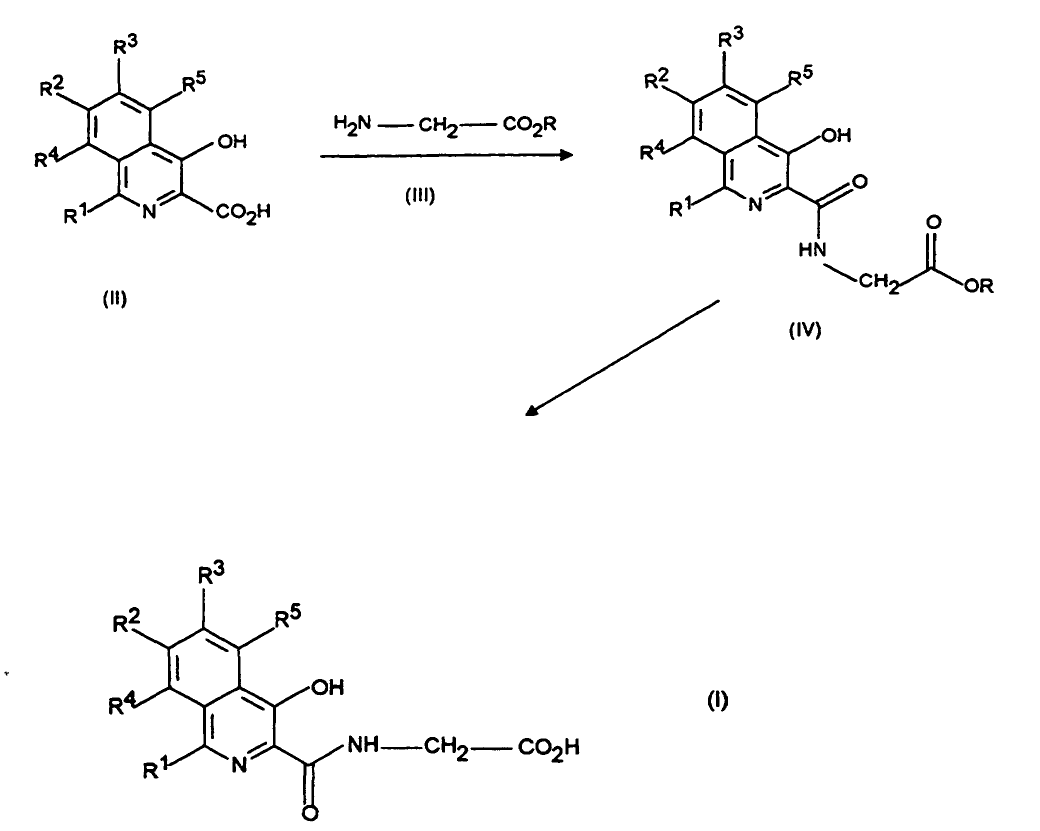
R = H, (C₁-C₈) Alkyl, Benzyl.
Die 4-Hydroxy-Gruppe der Verbindung der Formel II kann dabei auch geschützt vorliegen. Geeignete Schutzgruppen, (PG = Protecting Groups) wie sie dem Fachmann geläufig sind, sind insbesondere Benzyl, p-Methoxybenzyl, 3,4-Dimethoxybenzyl, Alkyl, Methoxymethyl (MOM), Methylthio, Benzyloxymethyl (BOM), t-Butyloxymethyl, 2-Methoxyethoxymethyl (MEM) und Tetrahydropyranyl (THP).

Weitere Schutzgruppen und die Bedingungen ihrer Abspaltung (Überführung von Verbindungen der Formel V in Verbindungen der Formel I) sind von Theodoro W. Greene, Peter G.M. Wuts, in Protective Groups in Organic Synthesis, Second Edition 1991, John Wiley, Kapitel 2 und 3, Seiten 10 bis 174 beschrieben.

Geeignete Verfahren zur Amidbildung (Umsetzung 1.i) sind die Methoden der Carboxylaktivierung und die aus der Peptidchemie bekannten Kondensationsreaktionen.

Als Reagenzien zur Carbonsäureaktivierung können die dem Fachmann bekannten Substanzen, wie Thionylchlorid, Oxalylchlorid, Pivaloylchlorid, Chlorameisensäureester-Derivate oder N,N'-Carbonyldimidazol Verwendung finden. Die aktivierten Derivate der Verbindungen der Formel II werden nach Herstellung in situ mit den Amidderivaten der Formel III umgesetzt.

Ein geeignetes Kondensationsmittel ist beispielsweise die Kombination von N,N'-Dicyclohexylcarbodiimid, 1-Hydroxy-1H-benzotriazol, (Benzotriazol-1-yloxy)-tripyrrolidinophosphonium-hexafluorophosphat (PyBOP) und N-Ethylmorpholin.

Geeignete Lösungsmittel sind Dichlormethan, Tetrachlormethan, Butylacetat, Ethylacetat, Toluol, Tetrahydrofuran, Dimethoxyethan, 1,4-Dioxan, Acetonitril, N,N-Dimethylformamid, N,N-Dimethylacetamid, Dimethylsulfoxid, Nitromethan und/oder Pyridin.

Substituierte 4-Hydroxyisochinolin-3-carbonsäuren bzw. deren Ester der Formeln II (IIa und IIb) sind aus entsprechenden 4-Hydroxy-1(2H)-isochinolon-3-carbonsäureestern der Formel V zugänglich. 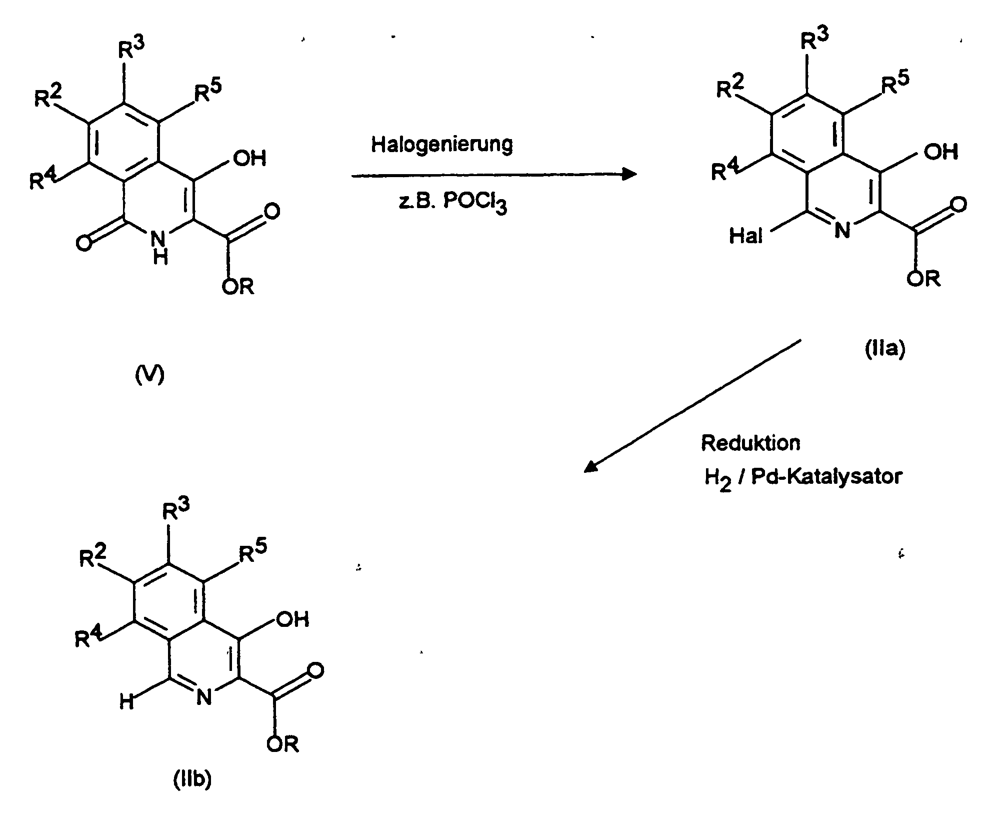 R = H, (C₁-C₈) Alkyl, Benzyl.
Die substituierten 4-Hydroxy-1(2H)-isochinolon-3-carbonsäureester der Formel V sind nach folgenden Methoden herstellbar:
1. Durch Umsetzung von Phthalsäureanhydriden mit Isocyanessigsäureestern/DBU und der sauren Isomerisierung der isolierten 1,3-Oxazole, wie in M. Suzuki et al., Synthesis 1978, 461 und K. Nunami, M. Suzuki. Chem. Pharm Bull 27, 1373 (1979) beschrieben, oder
2. Durch die Alkoholat-katalysierte Umlagerung von Phthalimidoacetaten der Formel VI zu Hydroxyisochinolonestern (Gabriel-Colman-Umlagerung), wie sie beispielsweise in L.R. Caswell, P.C. Atkinson, J. Heterocycl. Chem. 1966, 328; ibid, 1968, 865; ibid, 1973, 407 beschrieben ist. 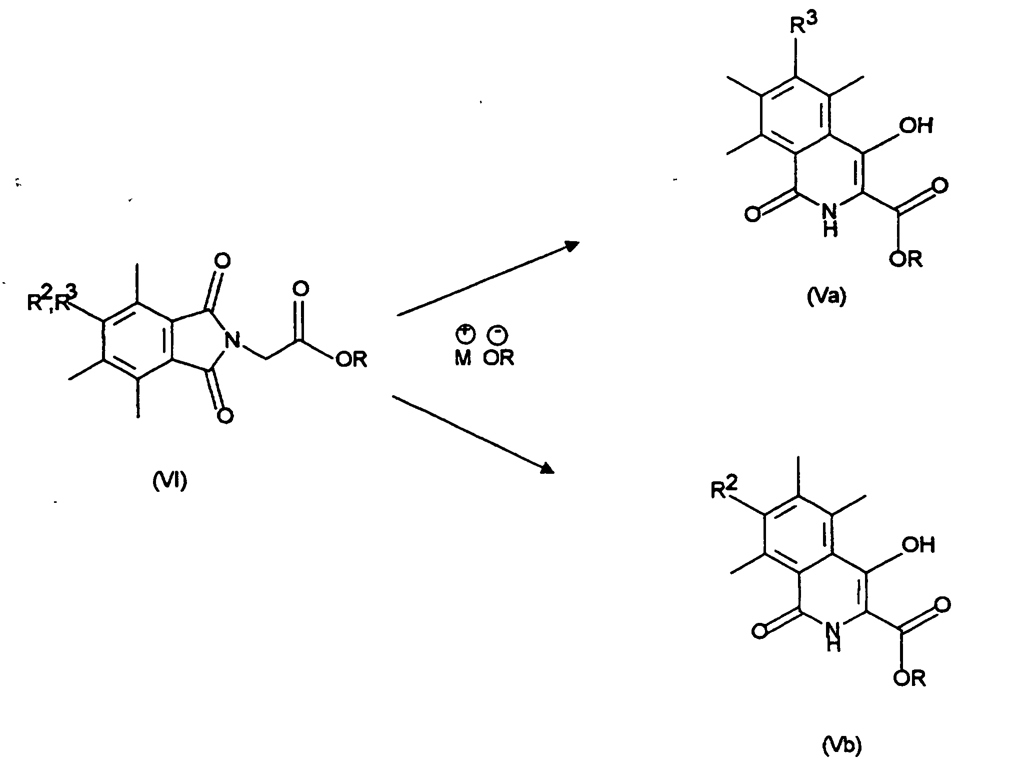 R = H, (C₁-C₈) Alkyl, Benzyl.
   Zur Herstellung der Verbindungen der Formel Ia werden die Verbindungen der Formeln IIa oder IIb mit 2-Aminoethanol umgesetzt.

Die Verbindungen der Formeln I und Ia sind Inhibitoren der Prolyl-4-hydroxylase. Die Hemmung dieses Enzyms wurde, wie von Kaule und Günzler in Annal. Biochem. 184, 291 bis 297 (1990) beschrieben, bestimmt.

| Beispiel Nr. | IC₅₀ [ Mol] |
|---|---|
| 1 | 0.12 |
| 2 | 1.90 |
| 3 | 0.79 |
| 4 | 0.57 |
| 5 | 0.70 |
| 6 | 2.02 |
| 7 | 1.34 |
| 8 | 0.62 |
| 9 | 2.30 |
| 10 | 3.60 |
| Referenz 11 | 0.66 |
| Referenz 12 | 0.65 |
| Referenz 13 | 9.30 |
| Referenz 14 | 0.35 |
| Referenz 15 | 0.66 |

Die erfindungsgemäßen Verbindungen der Formeln I und Ia besitzen weiterhin wertvolle pharmakologische Eigenschaften und zeigen insbesondere antifibrotische Wirksamkeit.

Die antifibrotische Wirkung kann im Modell der Tetrachlorkohlenstoffinduzierten Leberfibrose bestimmt werden. Dazu werden Ratten mit CCl₄ (1 ml/kg) - gelöst in Olivenöl - zweimal wöchentlich behandelt. Die Prüfsubstanz wird täglich, gegebenenfalls sogar zweimal täglich per os oder intraperitoneal - gelöst in einem geeigneten verträglichen Lösungsmittel - verabreicht. Das Ausmaß der Leberfibrose wird histologisch bestimmt und der Anteil Kollagen in der Leber per Hydroxyprolinbestimmung - wie bei Kivirikko et al. (Anal. Biochem. 19, 249 f. (1967)) beschrieben - analysiert. Die Aktivität der Fibrogenese kann durch radioimmunologische Bestimmung von Kollagenfragmenten und Prokollagenpeptiden im Serum bestimmt werden. Die erfindungsgemäßen Verbindungen sind in diesem Modell in Konzentration 1 bis 100 mg/kg wirksam.

### Kontroll-Tiere:

Im direkten Vergleich zu unbehandelten Tieren stieg der Hydroxyprolin-Gehalt der Leber durch Behandlung mit Tetrachlorkohlenstoff nach 4 Wochen (zweimalige Applikation pro Woche) um 200 % an. Die Messung der Hemmung der Hydroxyprolin-Bildung durch die Testsubstanzen wurde auf dieses Niveau bezogen.

### Substanz-Applikation:

Hierbei wurden die Versuchstiere 2 Wochen nur mit Tetrachlorkohlenstoff, sodann die nächsten 2 Wochen mit Tetrachlorkohlenstoff und Testsubstanz behandelt. Die Testsubstanzen wurden 2 x pro Tag in der angegebenen Gesamtmenge intraperitoneal appliziert.

### Ergebnisse:

| Testsubstanz aus Beispiel | Dosis [mg/kg] (Tierzahl) | Hemmung der Hydroxyprolin-Bildung |
|---|---|---|
| 1 | 20 (15) | - 55% |
| 9 | 20 (15) | - 67% |
| | 40 (15) | - 65% |
| | 100 (24) | - 58% |
| | 100* (14) | - 71% |
| Referenz 19 | 40 (13) | - 39% |

| | | |
|---|---|---|
| * orale Applikation | | |

Die Aktivität der Fibrogenese kann durch radioimmunologische Bestimmung des N-terminalen Propeptids des Kollagen Typ-III oder der N- bzw. C-terminalen Quervernetzungsdomäne des Kollagen-Typ-IV (7s-Kollagen bzw. Typ-IV-Kollagen NC₁) im Serum bestimmt werden.

Zu diesem Zweck wurden die Hydroxyprolin-, Prokollagen-III-Peptid-, 7s-Kollagen- und Typ-IV-Kollagen-NC-Konzentrationen in der Leber von
a) unbehandelten Ratten (Kontrolle)
b) Ratten, denen Tetrachlorkohlenstoff verabreicht wurden (CCl₄-Kontrolle)
c) Ratten, denen zunächst CCl₄ und anschließend eine erfindungsgemäße Verbindung verabreicht wurde
gemessen (diese Testmethode wird beschrieben von Rouiller, C., experimental toxic injury of the liver; in The Liver, C. Rouiller, Vol. 2, 5. 335 bis 476, New York, Academic Press, 1964).

Weiterhin kann eine Wirksamkeit der erfindungsgemäßen Verbindungen in folgenden Systemen nachgewiesen werden.

Hemmwirkung der Prolyl-4-hydroxylase-Inhibitoren im kombinierten Leberperfusions/Enzymassay:

Lebergewebe wandelt selektiv inaktive Proinhibitoren in inhibitorisch aktive Prolyl-4-hydroxylase-Hemmstoffe um, deren Wirkung sich hauptsächlich in diesem Organ entfaltet. Um die Hemmwirkung solcher aktiver Proinhibitoren zu untersuchen, wurden die Verbindungen in Perfusionsexperimenten an isolierter Rattenleber eingesetzt. Die Hemmwirkung wurde anschließend in einem Prolyl-4-hydroxylase-Assay gemessen.

### Methode:

Nicht gehungerte männliche Wistar-Ratten von 200-300 g Körpergeicht (Hoe:WISKf(SPF71)) wurden mit Pentobarbital betäubt (5 mg/100 g Körpergewicht i.p.) Nach Kanülieren der Portalvene wurde die Leber mit 100 ml heparinisierter Kochsalzlösung (5 IU/ml) von 37 C während 3 min. gewaschen. Die Flüssigkeit verließ die Leber durch die eingeschnittene V.cava. Anschließend wurde das Organ entnommen, an den Perfusionsapparat angeschlossen und mit 100 ml Medium 2 Stunden lang rezirkulierend bei einem Fluß von 30 ml/min perfundiert. Für die Perfusion wurde Krebs-Ringer-Puffer mit Rindererythrozyten verwendet. Dazu wurde Rinderblut mit einer Citratlösung 1:1 unmittelbar nach Entnahme im Schlachthaus gemischt. Diese Mischung wurde 10 min. bei 6000 U/min zentrifugiert und der Überstand entfernt. Dieser Vorgang wurde einmal mit Kochsalzlösung und zweimal mit Krebs-Ringer-Puffer wiederholt. Die endgültige Perfusionslösung enthielt 33.3% des Erythrozytensedimentes und 66.7% Krebs-Ringer-Puffer (Schimassek, H. Biochem. Z. 336 (1963) 460-467).

Zusammensetzung der verwendeten Lösungen:

| Citratlösung: | |
|---|---|
| Glucose Monohydrat | 22.6 g |
| Tri-Natriumcitrat | 4.0 g |
| Citronensäure | 5.5 g |
| NaCl | 4.2 g |
| ad 1000 ml mit destilliertem Wasser | |

| Krebs-Ringer-Puffer: | |
|---|---|
| NaCl | 8.0 g |
| KCl | 0.2 g |
| NaHCO₃ | 1.0 g |
| NaH₂PO₄ H₂O | 0.1 g |
| CaCl₂ | 0.2 g |
| MgCl₂ 6 H₂O | 0.1 g |
| Rinderserumalbumin | 16 g |
| ad 898 ml destilliertem Wasser, pH 7.4 | |

Perfusionsapparatur (Ryoo, H. und Tarver, H. P.S.E.B.M. 128 (1968) 760-772): Das zentrale Element der Apparatur ist ein thermostatisierter Zylinder mit einer herausnehmbaren Platte zur Lagerung des Organs. Der Auslaßschlauch wird verlängert und sein unteres Ende an eine peristaltische Pumpe angeschlossen. Vor der Rückkehr des Perfusats in das Organbad wird es durch eine Glasspirale, die als Wärmetauscher dient, welcher die Temperatur bei 37 C konstant hält, geleitet. Am Boden des Zylinders wird das Perfusat mit 70 ml einer CO₂/O₂-Mischung (5:95%) pro Minute begast. Um Schaumbildung zu vermeiden, werden pro ml Perfusionslösung 14 l 0.1 %igen Genapol PF-10-Lösung zugesetzt. Proben zur Analyse werden dem Perfusat an einer Stelle vor dem Eintritt in das perfundierte Organ entnommen.

### Behandlung:

Die Testsubstanzen wurden der Perfusionslösung zum Zeitpunkt t = 0 und 60 min. in Konzentrationen von jeweils 100 oder 50 g/ml zugefügt. Nach 120 min. Perfusionsdauer wurde der Perfusionslösung eine Probe zur Analyse entnommen.

### Enzymassay:

Die inhibitorische Aktivität der Metabolite, die während der zweistündigen Perfusion in der Leber entstanden, wurde in einem in vitro Assay der Prolyl-4-hydroxylase bestimmt. Das Enzym wird wie in der Literatur beschrieben aus 14 Tagen alten Hühnerembryonen gereinigt (Tuderman, L., Kuutti-Savolainen, E.R. und Kivirikko, K.I., Eur.J.Biochem. 52 (1975) 9-16; Kedersha, N.L. und Berg, R.A., Kollagen Rel. Res. 1 (1981) 345-353). Der Enzymtest wird nach Kaule und Günzler (Kaule G. und Günzler, V., Anal. Biochem. 184 (1990) 291-297) durchgeführt.Dosis-Wirkungskurven wurden durch Verdünnungsreihen, ausgehend von der unverdünnten Perfusionslösung erhalten.

### Ergebnisse:

| Beispiel Nr. | Hemmung der Prolyl-4-hydroxylase, IC₅₀ ( M) Vergleichssubstanz aus | |
|---|---|---|
| | Bespiel Nr. | Nach Perfusion |
| Referenz 19 | 1 | 10.5 |
| 20 | 2 | 16.6 |
| 21 | 3 | 7.5 |
| 22 | 4 | 6.4 |
| 23 | 5 | 21.7 |

### Hemmung der hepatischen Prolyl-4-hydroxylase in vivo:

Dieses Modell dient zum Nachweis der akuten Hemmung der Prolyl-4-hydroxylase in vivo. Dazu werden Ratten beiderlei Geschlechts (gesund bzw. mit induzierter Leberfibrose) die Prüfsubstanz bzw. das entsprechende Vehikel appliziert (intraperitoneal, intravenös, per os) und nach Substanzgabe ¹⁴C-L-Prolin (250 Ci/kg Körpergewicht) intraperitoneal verabreicht. Danach erfolgt erneut eine intraperitoneale Applikation von ¹⁴C-L-Prolin (250 Ci/kg Körpergewicht). Schließlich werden die Tiere unter Pentobarbitalnarkose entblutet und die Leber entnommen. Die Aufreinigung des hepatischen Kollagens durch Pepsinverdau und fraktionierte Ammoniumsulfatfällung erfolgte entsprechend publizierten Protokollen (Ref. 1, 2). Das gereinigte Leberkollagen wurde hydrolysiert und der Gehalt an ¹⁴C-Hydroxyprolin und ¹⁴C-Prolin durch Aminosäureanalyse mittels Ionenaustauschchromatografie bestimmt. Eine Hemmung der Prolyl-4-hydroxylase ergibt sich aus einer Absenkung des Quotienten ¹⁴C-Hydroxyprolin/[¹⁴C-Hydroxyprolin+¹⁴C-Prolin]. Als Referenzsubstanz wird 2,2'-Dipyridyl verwendet. (Ref. 1: Chojkier, M. 1986. Hepatocyte collagen production in vivo in normal rats. J. Clin. Invest. 78: 333-339 und Ref. 2: Ogata I., et al. 1991. Minor contribution of hepatocytes to collagen production in normal and early fibrotic livers. Hepatology 14: 361-367).

### Hemmung der Prolyl-4-hydroxylase in Zellkulturen:

Für die Testung von Prolyl-4-hydroxylasehemmstoffen in Zellkulturen werden folgende Zelltypen verwendet:
Normale humane Hautfibrolasten (Normal human fibrolasts, NHDF), und primäre Fettspeicherzellen aus der Rattenleber (fat storing cells, Ref. 2). Dazu werden die Zellen in Gegenwart von Hemmstoffen kultiviert. Gleichzeitig wird das in dieser Zeit neu synthetisierte Kollagen durch 4-³H-L-Prolin und ¹⁴C-Prolin metabolisch markiert. Der Einfluß der Testsubstanzen auf den Hydroxylierungsgrad des Kollagens wird anschließend entsprechend der Methode von Chojkier et al (Ref. 3) bestimmt. Als Referenzsubstanz wird 2,2'-Dipyridyl eingesetzt. 1. Blomhoff, R., Berg T. 1990. Isolation and cultivation of rat liver stellate cells. Methods Enzymol. 190: 59-71 und 2.: Chojkier, M. Peterkofsky, B. Bateman,, J. 1980. A new method for determining the extent of proline hydroxylation by measuring changes in the ration of [4-³H]:[¹⁴C] proline in collagenase digests. Anal. Biochem. 108: 385-393).

Bei der bis zu 6-wöchigen chronischen Behandlung männlicher Ratten (Fischer F344, Spraque Dawley, Wistar) mit der Verbindung des Beispiels 9 (N-((1-Chlor-4-hydroxyisochinolin-3-yl)carbonyl)glycin, gelöst in Wasser /NaHCO3, Gabe von 2 x 25 mg/kg i.p./Tag) konnte keine Leber-Steatose nachgewiesen werden.

Die Verbindungen der Formeln I und Ia können als Medikamente in Form pharmazeutischer Präparate Verwendung finden, welche sie gegebenenfalls mit verträglichen pharmazeutischen Trägern enthalten. Die Verbindungen können als Heilmittel, z.B. in Form pharmazeutischer Präparate Verwendung finden, welche diese Verbindungen in Mischung mit einem für die enterale, perkutane oder parenterale Applikation geeigneten pharmazeutischen, organischen oder anorganischen Träger, wie z.B. Wasser, Gummi arabicum, Gelatine, Milchzucker, Stärke, Magnesiumstearat, Talk, pflanzliche Öle, Polyalkylenglykole, Vaseline usw. enthalten.

Sie können zu diesem Zweck oral in Dosen von 0,1 bis 25 mg/kg/Tag, vorzugsweise 1 bis 5,0 mg/kg/Tag oder parenteral in Dosen von 0,01 bis 5 mg/kg/Tag, vorzugsweise 0,01 bis 2,5 mg/kg/Tag, insbesondere 0,5 bis 1,0 mg/kg/Tag, appliziert werden. Die Dosierung kann in schweren Fällen auch erhöht werden. In vielen Fällen genügen jedoch auch geringere Dosen. Diese Angaben beziehen sich auf einen Erwachsenen von etwa 75 kg Gewicht.

Unter den im folgenden beschriebenen Beispielen werden die erfindungsgemäßen Verbindungen der Formel I als substituierte N-((Isochinolin-3-yl)-carbonyl)glycine bezeichnet. Die Bezeichnung als substituierte Isochinolin-3-carbonsäure-N-(carboxymethyl)amide (Isochinolin-3-carbonsäure-glycinamide) ist ebenso möglich.

### Beispiele

### Beispiel 1

### N-((1-Chlor-4-hydroxy-7-((2-propyl)oxy)isochinolin-3-yl)carbonyl)glycin

a) 4-(2-Propyloxy)phthalsäurediethylester
   54 g 4-Hydroxyphthalsäure wurden in 1,2 l Ethanol und 25 ml conc. Schwefelsäure 4 h unter Rückfluß verestert. Man erhielt 60 g (0,252 Mol) Diethylester der anschließend in 250 ml N,N-Dimethylacetamid mit 37,3 g (0,27 Mol) gepulvertem Kaliumcarbonat 30 min bei 80 C gerührt wurde. Bei 40 C wurden 25,7 ml (0,26 Mol) 2-Propyljodid zugetropft und 90 min bei 100 C gerührt. Nach dem Abkühlen wurde im Vakuum eingeengt, der Rückstand in Wasser mit 2 N Salzsäure auf pH 7 gebracht und zweimal mit Ethylacetat extrahiert. Nach dem Trocknen über Magnesiumsulfat und Einengen erhielt man 65 g öliges Rohprodukt.
b) 4-(2-Propyloxy)phthalsäure
   65 g des vorstehenden Diesters wurden bei 20 C in 400 ml 3N ethanolische KOH eingetragen und 1 h bei 45 C gerührt. Das ausgefallene K-Salz wurde abgesaugt, in Wasser gelöst, unter Kühlung mit conc. wäßriger Salzsäure auf pH 1 gebracht und zweimal mit Ethylacetat extrahiert. Nach dem Trocknen und Einengen der organischen Phase erhielt man 44 g der Titelverbindung, Fp. 116-118 C.
c) 4-((2-Propyloxy)phthaloyl)imino-essigsäure
   44 g (0,2 Mol) der vorstehenden Dicarbonsäure wurden in 250 ml Dowtherm mit 16 g (0,2 Mol) Glycin 1 h bei 200 C Badtemperatur im offenen Kolben gerührt. Nach dem Abkühlen wurde mit 800 ml Petrolether versetzt, ausgefallenes Harz in 400 ml gesättigter Natriumbicarbonat-Lösung aufgenommen, zweimal mit Ethylacetat extrahiert, die wäßrige Phase mit 2N wäßriger Salzsäure auf pH 1 gebracht, zweimal mit Dichlormethan extrahiert, die organische Phase getrocknet und eingeengt.
   Man erhielt 47 g Produkt, Fp. 120 - 122 C.
d) 4-((2-Propyloxy)phthaloyl)iminoessigsäure-(1-butyl)ester
   47 g des vorstehenden Produkts wurden in 600 ml 1-Butanol mit 9 ml conc. Schwefelsäure 1 h bei Rückfluß gerührt. Nach dem Abkühlen wurde im Vakuum eingeengt. Der Rückstand in 300 ml Ethylacetat gelöst, diese Lösung mit wäßriger Natriumbicarbonat Lösung geschüttelt, die organische Phase getrocknet, eingeengt und der Rückstand (54 g) mit Ethylacetat (n-Heptan (1:5) an Kieselgel chromatographiert.
   Man erhielt 42,4 g Produkt, Fp. 83-85 C.
e) 4-Hydroxy-7-(2-propyloxy)-1(2H)-isochinolon-3-carbonsäure-(1-butyl)ester (A) und 4-Hydroxy-6-(2-propyloxy)-1(2H)isochinolon-3-carbonsäure-(1-butyl)ester (B)
   Unter Stickstoffatmosphäre wurden 3,2 g (140 mMol) Natrium bei 60 - 80 C unter Rühren in 350 ml 1-Butanol gelöst. Bei 20 C wurden dann 22,3 g (70 mMol) der vorstehenden Verbindung zugegeben und 45 min bei 95 C gerührt. Die Reaktionslösung färbte sich von farblos nach schwarz, dann nach grün. Nach dem Abkühlen wurde die Lösung in 300 ml 2N Salzsäure eingerührt, abgesaugt, der Rückstand mit 150 ml Diethylether behandelt. Man erhielt 11,5 g des Produkts (B), Fp. 168 - 170 C. Aus der Mutterlauge erhielt man 1,2 g des Produkts A. Die Butanol-Phase wurde getrocknet, im Vakuum eingeengt und der erhaltene Rückstand mit Diethylether zur Kristallisation gebracht.
   Man erhielt 5,2 g des Produkts A, Fp. 118-123 C.
   Eine Unterscheidung der Isomeren A und B gelingt auch im DC mit Ethylacetat an Kieselgel: A: R_{f} ca. 0,5; B: R_{f} ca. 0,35.
f) 1-Chlor-4-hydroxy-7-(2-propyloxy)isochinolin-3-carbonsäure-(1-butyl)ester
   12 g der obigen Verbindung wurden 30 min in 120 ml Phosphoroxychlorid zum Sieden erhitzt. Nach Aufarbeitung und Chromatographie mit Ethylacetat/Heptan (1:5) an Kieselgel: 7,3 g Produkt, Fp. 60 - 62 C.
g) 1-Chlor-4-hydroxy-7-(2-propyloxy)-isochinolin-3-carbonsäure
   3,7 g (11 mMol) des vorstehenden Esters wurden in 150 ml 2N Natronlauge/Ethanol (1:1) 1 h rückfließend erhitzt. Man engte im Vakuum ein, säuerte an, versetzte mit Tetrahydrofuran bis die Lösung klar wurde, engte im Vakuum ein und saugte 3,0 g des ausgefallenen Produktes ab, Fp. 139 - 141 C.
h) N-((1-Chlor-4-hydroxy-7-(2-propyloxy)isochinolin-3-yl)carbonyl)glycin-(1-pentyl)ester
   4,8 g (17 mMol) der vorstehenden Isochinolincarbonsäure wurden in 600 ml Dichlormethan mit 8,9 ml (70 mMol) N-Ethylmorpholin (NEM), 6,3 g (20 mMol) Glycin-(1-pentyl)ester-Tosylat, 2,7 g (20 mMol) 1-Hydroxy-1H-Benztriazol (HOBT) und 8,5 g (20 mMol) N-Cyclohexyl-N -(2-morpholinoethyl)-carbodiimid-methyl-p-toluolsulfonat (CMC) versetzt und 8 Tage bei 20 C gerührt.
   Dann wurde im Vakuum eingeengt, der Rückstand in Ethylacetat gelöst, mit wäßriger Natriumbicarbonat-Lösung extrahiert. Die Ethylacetat-Phase wurde mit 2N wäßriger Salzsäure, dann mit Wasser geschüttelt, getrocknet und eingeengt.
   Man erhielt 3,5 g Produkt, Fp. 70 - 72 C. Nach Behandeln mit Petrolether, Fp. 73 - 75 C.
i) Die Titelverbindung wurde wie folgt erhalten:
   4,6 g (11 mMol) des vorstehenden Glycinesters wurden 1 h in 100 ml 1,5 N methanolischer Natronlauge gerührt, wobei sich nach 15 min ein dicker Niederschlag bildete. Dann wurde im Vakuum eingeengt, der Rückstand in Wasser gelöst, einmal mit Diethylether geschüttelt, mit Aktivkohle geklärt und unter Kühlung mit wäßriger Salzsäure angesäuert.
   Man erhielt 3,5 g des farblosen Produktes, Fp. 207 - 208 C.

### Beispiel 2

### N-((1-Chlor-4-hydroxy-6-((2-propyl)oxy)isochinolin-3-yl)carbonyl)glycin

a) 1-Chlor-4-hydroxy-6-(2-propyloxy)isochinolin-3-carbonsäure-(1-butyl)ester
   2,0 g der Verbindung B aus Beispiel 1e) wurden analog 1f) mit Phopsphoroxychlorid umgesetzt. Nach Chromatographie mit Heptan/Ethylacetat (4:1) an Kieselgel wurden 1,5 g Produkt isoliert, Fp. 116-118 C (aus Petrolether).
b) 1-Chlor-4-hydroxy-6-(2-propyloxy)isochinolin-3-carbonsäure
   3,3 g des vorstehenden Esters wurden analog Beispiel 1g) verseift. Man erhielt 2,8 g Produkt, Fp. 186-188 C (aus wäßriger Salzsäure/Tetrahydrofuran).
c) N-((1-Chlor-4-hydroxy-6-(2-propyloxy)isochinolin-3-yl)carbonyl)glycin-(1-pentyl)ester
   2,8 g (10 mMol) der vorstehenden Carbonsäure wurden analog Beispiel 1h) 48 h mit 3,2 g (10 mMol) Glycin-(1-pentyl)ester-Tosylat, 6 ml (40 mMol) NEM, 1,35 g (10 mMol) HOBT und 42,4 g (10 mMol) CMC umgesetzt.
   Man erhielt 1,33 g Produkt, Fp. 75-77 C (aus Petrolether).
d) Die Titelverbindung wurde erhalten, indem 0,45 g des vorstehenden Glycinesters bei 20 C in 50 ml 1,5 N methanolischer Natronlauge verseift wurden. Nach dem Einengen wurde nach Versetzen mit wäßriger Salzsäure das Produkt zur Kristallisation gebracht.
   Man erhielt 0,37 g Produkt, Fp. 223 - 225 C.

Die Verbindungen der nachfolgenden Beispiele 3 bis 8 wurden analog den Beispielen 1 bzw. 2 erhalten.

### Beispiel 3

### N-((1-Chlor-4-hydroxy-7-methoxyisochinolin-3-yl)carbonyl)glycin

a) 4-Methoxyphthaloyliminoessigsäure-(1-butyl)ester
   Fp. 63 - 64 C (aus Petrolether)
b) 4-Hydroxy-7-methoxy-1(2H)-isochinolon-3-carbonsäure-(1-butyl)ester
   Fp. 125 - 127 C (aus Diethylether). Gehalt ca. 90 %.
c) 1-Chlor-4-hydroxy-7-methoxyisochinolin-3-carbonsäure-(1-butyl)ester
   Fp. 112 C (aus Petrolether)
d) 1-Chlor-4-hydroxy-7-methoxyisochinolin-3-carbonsäure
   Fp. 185 C (aus wäßriger Salzsäure, Tetrahydrofuran)
e) N-((1-Chlor-4-hydroxy-7-methoxyisochinolin-3-yl)carbonyl)glycin-(1-pentyl)ester
   Fp. 93 - 94 C (aus Petrolether)
f) Die Titelverbindung wurde durch Verseifung des vorstehenden Glycinesters erhalten
   Fp. 231 C (aus wäßriger Salzsäure/Tetrahydrofuran).

### Beispiel 4

### N-((1-Chlor-4-hydroxy-6-methoxyisochinolin-3-yl)carbonyl)glycin

a) 4-Hydroxy-6-methoxy-1(2H)-isochinolon-3-carbonsäure-(1-butyl)ester
   Fp. 193 - 195 C (aus wäßriger Salzsäure/1-Butanol)
b) 1-Chlor-4-hydroxy-6-methoxyisochinolin-3-carbonsäure-(1-butyl)ester
   Fp. 114 - 116 C (aus Petrolether)
c) 1-Chlor-4-hydroxy-6-methoxyisochinolin-3-carbonsäure
   Fp. 174 - 176 C (aus wäßriger Salzsäure/Tetrahydrofuran)
d) N-((1-Chlor-4-hydroxy-6-methoxyisochinolin-3-yl)carbonyl)glycin-(1-pentyl)ester
   Fp. 109 - 111 C (aus Petrolether)
e) Die Titelverbindung wurde durch Verseifung des obigen Glycinesters erhalten
   Fp. 212 - 214 C (aus wäßriger Salzsäure/Tetrahydrofuran).

### Beispiel 5

### N-((7-((1-Butyl)oxy)-1-chlor-4-hydroxy-isochinolin-3-yl)carbonyl)glycin

a) 4-((1-Butyloxy)phthalsäure
b) 4-((1-Butyloxy)phthaloylimino)essigsäure,
c) 4-((1-Butyloxy)phthaloylimino)essigsäure-(1-butyl)ester
d) 7-(1-Butyloxy)-4-hydroxy-1(2H)-isochinolon-3-carbonsäure-(1-butyl)ester
   Fp. 133 - 135 C (aus n-Heptan/Ethylacetat (3:2)
e) 7-(1-Butyloxy)-1-chlor-4-hydroxyisochinolin-3-carbonsäure-(1-butyl)ester
f) 7-(1-Butyloxy)-1-chlor-4-hydroxyisochinolin-3-carbonsäure
   Fp. 140 - 142 C (aus wäßriger Salzsäure/Tetrahydrofuran)
g) N-((7-(1-Butyloxy)-1-chlor-4-hydroxyisochinolin-3-yl)carbonyl)glycin-(1-butyl)ester
   Fp. 78 - 80 C (aus Petrolether)
h) die Titelverbindung wurde aus der Verseifung des obigen Glycinesters erhalten
   Fp. 158 - 160 C (aus Ethylacetat).

### Beispiel 6

### N-((-6-((1-Butyl)oxy-1-chlor-4-hydroxy-isochinolin-3-yl)carbonyl)glycin

a) 6-(1-Butyloxy)-4-hydroxy-1(2H)-isochinolon-3-carbonsäure-(1-butyl)ester
   Fp. 160 - 162 C (aus n-Heptan/Ethylacetat (1:1)
b) 6-(1-Butyloxy)-1-chlor-4-hydroxyisochinolin-3-carbonsäure-(1-butylester)
   Fp. 76 - 78 C (aus n-Heptan/Ethylacetat (1:1))
c) 6-(1-Butyloxy)-1-chlor-4-hydroxyisochinolin-3-carbonsäure
   Fp. 112 - 113 C (aus Tetrahydrofuran/Ethanol)
d) N-((6-(1-Butyloxy)-1-chlor-4-hydroxy-isochinolin-3-yl)carbonyl)glycinbenzyl-ester
e) Titelverbindung
   Fp. 182 - 184 C (aus wäßriger Salzsäure/Tetrahydrofuran).

### Beispiel 7

### N-((6-Benzyloxy-1-chlor-4-hydroxy-isochinolin-3-yl)carbonyl)glycin

a) 4-Benzyloxyphthaloyliminoessigsäure-(1-butyl)ester
   Fp. 59 - 61 C (aus n-Heptan/Ethylacetat (1:1))
b) 6-Benzyloxy-4-hydroxy-1(2H)-isochinolon-3-carbonsäure-(1-butyl)ester
   Fp. 193 - 195 C (aus Butanol/Ethylacetat)
c) 6-Benzyloxy-1-chlor-4-hydroxy-isochinolin-3-carbonsäure-(1-butyl)ester
d) 6-Benzyloxy-1-chlor-4-hydroxyisochinolin-3-carbonsäure
   Fp. 203 - 205 C (aus wäßriger Salzsäure/Tetrahydrofuran)
e) N-((6-Benzyloxy-1-chlor-4-hydroxyisochinolin-3-yl)carbonyl)glycin-ethylester
   Fp. 124 - 127 C (aus Diisopropylether)
f) Titelverbindung
   Fp. 210 - 211 C (aus Diethylether).

### Beispiel 8

### N-((7-Benzyloxy-1-chlor-4-hydroxy-isochinolin-3-yl)carbonyl)glycin

a) 7-Benzyloxy-4-hydroxy-1 (2H)-isochinolon-3-carbonsäure-(1-butyl)ester
b) 7-Benzyloxy-1-chlor-4-hydroxyisochinolin-3-carbonsäure-(1-butyl)ester
   Fp. 115 - 117 C (aus Petrolether)
c) 7-Benzyloxy-1-chlor-4-hydroxy-isochinolin-3-carbonsäure
   Fp. 166 - 168 C (aus wäßriger Salzsäure/Tetrahydrofuran)
d) N-((7-Benzyloxy-1-chlor-4-hydroxyisochinolin-3-yl)carbonyl)glycin-(1-pentyl)ester
   Fp. 121 - 123 C (aus Diisopropylether)
e) Titelverbindung
   Fp. 194 - 196 C (aus wäßriger Salzsäure/Tetrahydrofuran).

### Beispiel 9

### N-((1-Chlor-4-hydroxyisochinolin-3-yl)carbonyl)glycin

a) 1-Chlor-4-hydroxyisochinolin-3-carbonsäuremethylester
   2,84 g (13 mMol) 4-Hydroxy-1(2H)-isochinolon-3-carbonsäuremethylester (hergestellt wie in M. Suzuki, Synthesis 1978, 461 beschrieben) wurden in 25 ml Phosphoroxychlorid 3 h bei 70 C gerührt. Nach dem Abkühlen wurde auf 500 ml Eiswasser gegeben, der Niederschlag am nächsten Morgen abgesaugt und am IR-Strahler bei 70 C getrocknet. Man erhielt 2,96 g Produkt, Fp. 168 C.
b) 1-Chlor-4-hydroxyisochinolin-3-carbonsäure
   41,0 g (0,17 mMol) des vorstehenden Esters wurden in 500 ml Ethanol und 500 ml 2N wäßriger Natronlauge 5 h bei 90 C gerührt. Nach Ansäuern mit wäßriger Salzsäure auf pH 2 erhielt man 38,9 g Produkt,
   Fp. 192 C (Zersetzung).
c) N-((1-Chlor-4-hydroxyisochinolin-3-yl)carbonyl)glycinmethylester
   1,12 g (5,0 mMol) der vorstehenden Carbonsäure und 0,63 g (5,0 mMol) Glycinmethylester-Hydrochlorid wurden in wasserfreiem Dichlormethan mit 0,7 ml Triethylamin, 2,60 g (Benzotriazol-1-yloxy)-tripyrrolidinophosphonium-hexafluorophosphat (PyBOP) und 1,7 ml Ethyldiisopropylamin versetzt und 3 h bei 20 C gerührt. Nach Abfiltrierten von Ungelöstem wurde dreimal mit Wasser gewaschen, die organische Phase getrocknet, eingeengt und der Rückstand mit Dichlormethan an Kieselgel gereinigt.
   Man erhielt 0,72 g Produkt, Fp. 129 C.
d) Die Titelverbindung wurde erhalten, indem 14,1 g (48,1 mMol) des vorstehenden Esters in 100 ml Tetrahydrofuran gelöst mit 100 ml 1N Natronlauge 2 h bei 20 C gerührt wurden. Dann wurde im Vakuum eingeengt, mit Wasser verdünnt, dreimal mit Dichlormethan extrahiert, die wäßrige Phase mit conc. Salzsäure auf pH 3 gebracht, das ausgefallene Produkt abgesaugt und getrocknet. Man erhielt 12,38 g Produkt, Fp. 213 C (Zersetzung).

### Beispiel 10

### N-((1,6,7-Trichlor-4-hydroxyisochinolin-3-yl)carbonyl)glycin (wurde analog M. Suzuki et al., Synthesis 1978, 461 bzw. K.Nunami, M. Suzuki, chem. Pharm Bull 27, 1373 (1979) erhalten)

a) 6,7-Dichlor-4-hydroxy-1(2H)-isochinolon-3-carbonsäuremethylester
   Fp. 295 C (u. Zersetzung, aus Methanol)
b) 1,6,7-Trichlor-4-hydroxyisochinolin-3-carbonsäuremethylester
   Fp. 246 - 248 C (aus Wasser)
c) 1,6,7-Trichlor-4-hydroxyisochinolin-3-carbonsäure
   Fp. 200 C (u. Zersetzung, aus wäßriger Salzsäure/Tetrahydrofuran)
d) N-((1,6,7-Trichlor-4-hydroxy-isochinolin-3-yl)carbonyl)glycin-(1-butyl)ester
   Fp. 156 - 158 C (aus Diisopropylether)
e) Titelverbindung
   Fp. 265 C (u. Zersetzung, aus wäßriger Salzsäure).

### Beispiel 11 (nicht erfindungsgemäß)

### N-((8-Chlor-4-hydroxyisochinolin-3-yl)carbonyl)glycin

a) 8-Chlor-4-hydroxyisochinolin-3-carbonsäure
   Fp. 209 C (aus wäßriger Salzsäure)
b) N-((8-Chlor-4-hydroxyisochinolin-3-yl)carbonyl)glycin-methylester
   Fp. 103 C (aus Ethylacetat/n-Heptan (1:1))
c) Titelverbindung
   Fp. 232 C (aus Wasser).

### Beispiel 12 (nicht erfindungsgemäß)

### N-((4-Hydroxy-8-methoxyisochinolin-3-yl)carbonyl)glycin

a) 4-Hydroxy-8-methoxyisochinolin-3-carbonsäure
   Fp. 217 C (aus wäßriger Salzsäure)
b) Titelverbindung
   Fp. 168 C (aus Wasser).

### Beispiel 13 (nicht erfindungsgemäß)

### N-((7-((1-Butyl)oxy)-4-hydroxyisochinolin-3-yl)carbonyl)glycin

a) 7-(1-Butyloxy)-4-hydroxyisochinolin-3-yl)carbonsäure-(1-butyl)ester
   Die Verbindung aus Beispiel 5e) wurde zunächst in Tetrahydrofuran mit Pd und Wasserstoff hydriert. Eine vollständige Umsetzung gelingt in Methanol plus 5 % Ameisensäure mit Pd/C; öliges Rohprodukt.
b) 7-(1-Butyloxy)-4-hydroxyisochinolin-3-carbonsäure
   Fp. 180 C (u. Zersetzung, aus wäßriger Salzsäure/Tetrahydrofuran)
c) N-((7-(1-Butyloxy)-4-hydroxyisochinolin-3-yl)carbonyl)glycin-(1-butyl)ester öliges Rohprodukt
d) Titelverbindung
   Fp. 151 - 153 C (aus wäßriger Salzsäure).

### Beispiel 14 (nicht erfindungsgemäß)

### N-((6-((1-Butyl)oxy)-4-hydroxy-isochinolin-3-yl)carbonyl)glycin

a) 6-(1-Butyloxy)-4-hydroxyisochinolin-3-carbonsäure-(1-butyl)ester wurde durch Hydrierung der Verbindung aus Beispiel 6b) erhalten; öliges Rohprodukt.
b) 6-(1-Butyloxy)-4-hydroxyisochinolin-3-carbonsäure
   Fp. 185 - 187 C (aus wäßriger Salzsäure/Tetrahydrofuran)
c) N-((6-(1-Butyloxy)-4-hydroxyisochinolin-3-yl)carbonyl)glycinbenzylester
   Fp. 98 - 100 C (aus N-Heptan/Ethylacetat(1:1))
d) Die Titelverbindung wurde durch Hydrierung des vorstehenden Benzylethers erhalten.
   Fp. 199 - 200 C (aus Petrolether).

### Beispiel 15 (nicht erfindungsgemäß)

### N-((4-Hydroxy-7-((2-propyl)oxy)isochinolin-3-yl)carbonyl)glycin

Analog der Verbindung des Beispiels 1 können die Verbindungen der Beispiele 16 bis 18 erhalten werden.

### Beispiel 16 (nicht erfindungsgemäß)

### N-((4-Hydroxy-7-(3-pentyloxy)isochinolin-3-yl)carbonyl)glycin

### Beispiel 17 (nicht erfindungsgemäß)

### N-((4-Hydroxy-7-trifluormethoxy-isochinolin-3-yl)carbonyl)glycin

### Beispiel 18 (nicht erfindungsgemäß)

### N-((7-Difluormethoxy-4-hydroxyisochinolin-3-yl)carbonyl)glycin

### Beispiel 19 (nicht erfindungsgemäß)

### 1-Chlor-4-hydroxy-7-(2-propyloxy)isochinolin-3-carbonsäure-N-(2-hydroxyethyl)amid

0,3 g der Verbindung aus Beispiel 1f wurden im 5 ml 2-Aminoethanol 1 h bei 85 C gerührt. Dann wurde mit 30 ml Wasser versetzt, mit halbkonzentrierter, wäßriger Salzsäure unter Kühlung auf pH 1 gestellt, zweimal mit Ethylacetat extrahiert, getrocknet, eingeengt und der Rückstand mit wenig Diisopropylether/Petrolether (1:1) zur Kristallisation gebracht.
Man erhielt 0,21 g der Titelverbindung, Fp. 102-104 C.

### Beispiel 20

### 1-Chlor-4-hydroxy-6-(2-propyloxy)isochinolin-3-carbonsäure-N-(2-hydroxyethyl)amid

Die Titelverbindung wurde ausgehend von der Verbindung aus Beispiel 2a) und 2-Aminoethanol analog Beispiel 19 hergestellt.
Fp. 155-156 C (aus Petrolether).

### Beispiel 21

### 1-Chlor-4-hydroxy-7-methoxyisochinolin-3-carbonsäure-N-(2-hydroxyethyl)amid

Die Titelverbindung wurde ausgehend von der Verbindung aus Beispiel 3c) und 2-Aminoethanol analog Beispiel 19 hergestellt.
Fp. 165-167 C (aus Diisopropylether).

### Beispiel 22

### 1-Chlor-4-hydroxy-6-methoxyisochinolin-3-carbonsäure-N-(2-hydroxyethyl)amid

Die Titelverbindung wurde ausgehend von der Verbindung aus Beispiel 4a) und 2-Aminoethanol analog Beispiel 19 hergestellt.
Fp. 117-119 C (aus Diisopropylether).

### Beispiel 23

### 7-(1-Butyloxy)-1-chlor-4-hydroxyisochinolin-3-carbonsäure-N-(2-hydroxyethyl)amid

Die Titelverbindung wurde ausgehend von der Verbindung aus Beispiel 5e) und 2-Aminoethanol analog Beispiel 19 hergestellt.
Fp. 118-120 C (aus Diisopropylether).

## Patentansprüche

1. Verbindungen der Formel I 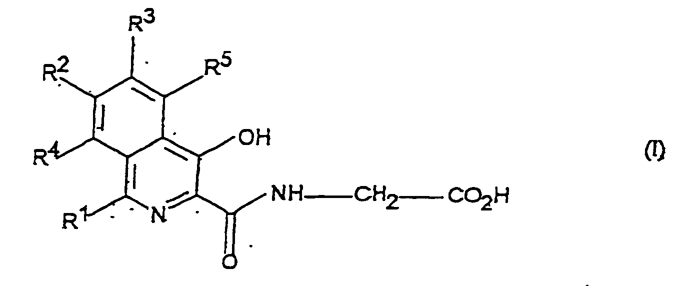 in welcher bedeuten
R¹ Chlor
R² Wasserstoff, (C₁-C₈)-Alkyl, (C₁-C₈)-Alkoxy, Chlor, Trifluormethyl, Hydroxy, oder Benzyloxy, das gegebenenfalls mit Substituenten aus der Reihe (C₁-C₅)-Alkyl, (C₁-C₅)-Alkoxy substituiert ist, oder Fluoralkoxy der Formel
O-[CH₂]ₓ-C_{f}H_{(2f+1-g)}F_{g}
mit
x = 0 oder 1
f = 1 - 5 und
g = 1 bis (2f + 1),
R³ Wasserstoff, (C₁-C₈)-Alkyl, (C₁-C₈)-Alkoxy, Fluor, Chlor, Cyano, Trifluormethyl, Hydroxy, oder Benzyloxy, das gegebenenfalls mit Substituenten aus der Reihe (C₁-C₅)-Alkyl, (C₁-C₅)-Alkoxy substituiert ist, oder Fluoralkoxy der Formel
O-[CH₂]ₓ-C_{f}H_{(2f+1-g)}F_{g}
worin x, f, und g wie oben definiert sind,
R⁴ und R⁵ Wasserstoff, (C₁-C₅)-Alkyl, Fluor, Chlor, Brom, Trifluormethyl, Cyano, (C₁-C₅)-Alkoxy oder Fluoralkoxy der Formel
O-[CH₂]ₓ-C_{f}H_{(2f+1-g)}F_{g}
worin x, f und g wie oben definiert sind,
einschließlich der physiologisch wirksamen Salze.

2. Verbindungen der Formel I gemäß Anspruch 1, in welcher
R ¹ Chlor,
R² Wasserstoff, (C₁-C₈)-Alkyl, (C₁-C₈)-Alkoxy, Chlor, Trifluormethyl, Hydroxy, Benzyloxy oder Fluoralkoxy der Formel
O-[CH₂]ₓ-C_{f}H_{(2f + 1-g)}F_{g}
mit
x² = 0 oder 1
f = 1 - 5 und
g = 1 bis (2f + 1),
R³ Wasserstoff, (C₁-C₈)-Alkyl, (C₁-C₈)-Alkoxy, Fluor Chlor, Cyano, Trifluormethyl, Hydroxy, Benzyloxy, oder Fluoralkoxy der Formel
O-[CH₂]ₓ-C_{f}H_{(2f + 1-g)}F_{g}
worin x, f und g wie vorstehend definiert sind,
R⁴ Wasserstoff, (C₁-C₈)-Alkoxy, Fluor, Chlor, Trifluormethyl, Cyano oder Fluoralkoxy der Formel
O-[CH₂]ₓ-C_{f}H_{(2f + 1-g)}F_{g}
worin x, f und g wie vorstehend definiert sind, und
R⁵ Wasserstoff sind.

3. Verbindungen der Formel I gemäß Anspruch 1 oder 2, in welcher
R¹ Chlor,
R² Wasserstoff, (C₁-C₅)-Alkoxy, Chlor, Benzyloxy, Fluoralkoxy mit x = 0, f = 1,
R³ Wasserstoff, (C₁-C₅)-Alkoxy, Chlor, Benzyloxy,
R⁴ Wasserstoff, Chlor, Methoxy,
R⁵ Wasserstoff sind.

4. Verbindungen der Formel I gemäß Anspruch 1 oder 2, in welcher
R¹ Chlor,
R² Wasserstoff, (C₁-C₈)-Alkoxy, Chlor, Hydroxy, Benzyloxy,
R³ Wasserstoff, (C₁-C₈)-Alkoxy, Fluor, Chlor, Hydroxy, Benzyloxy,
R⁴ Wasserstoff, Chlor,
R⁵ Wasserstoff sind.

5. Verbindungen der Formel I gemäß Anspruch 1 oder 2, in welcher
R¹ Chlor,
R² (C₁-C₈)-Alkoxy, Chlor, Benzyloxy und
R³, R⁴ und R⁵ Wasserstoff sind.

6. Verbindungen der Formel I gemäß Anspruch 1 oder 2, in welcher
R¹ Chlor,
R² Wasserstoff oder Chlor,
R³ (C₁-C₈)-Alkoxy, Chlor, Benzyloxy, und
R⁴ und R⁵ Wasserstoff sind.

7. Verbindungen der Formel I gemäß Anspruch 1 oder 2, in welcher
R¹ Chlor,
R² und Wasserstoff oder Chlor,
R⁴ (C₁-C₆)-Alkoxy, Chlor, Benzyloxy und
R⁵ Wasserstoff sind.

8. Verbindungen der Formel I gemäß Anspruch 1 oder 2, in welcher
R¹ Chlor,
R² (C₁-C₆)-Alkoxy oder Benzyloxy,
R³, R⁴ und R⁵ Wasserstoff sind.

9. Verbindungen der Formel I gemäß Anspruch 1 oder 2, in welcher
R¹ Chlor,
R² Wasserstoff
R³ (C₁-C₆)-Alkoxy oder Benzyloxy, und
R⁴ und R⁵ Wasserstoff sind.

10. Verbindungen der Formel I gemäß Anspruch 1 oder 2, in welcher
R¹ Chlor,
R² (C₁-C₆)-Alkoxy und
R³, R⁴ und R⁵ Wasserstoff sind.

11. N-((1-Chlor-4-hydroxyisochinolin-3-yl)carbonyl)glycin, N-((1-Chlor-4-hydroxy-7-((2-propyl)oxy)isochinolin-3-yl)carbonyl)glycin.

12. Verfahren zur Herstellung von Verbindungen der allgemeinen Formel I gemäß den Ansprüchen 1 bis 11, **dadurch gekennzeichnet, dass**
1.i) Chinolin-2-carbonsäuren der Formel II mit den Aminoestern der Formel III zu den Amidestern der Formel IV umgesetzt werden, wobei R Wasserstoff, (C₁-C₈)-Alkyl oder Benzyl bedeutet, und
1.ii) die. Verbindungen der Formel I aus ihren Estern der Formel IV freigesetzt werden.

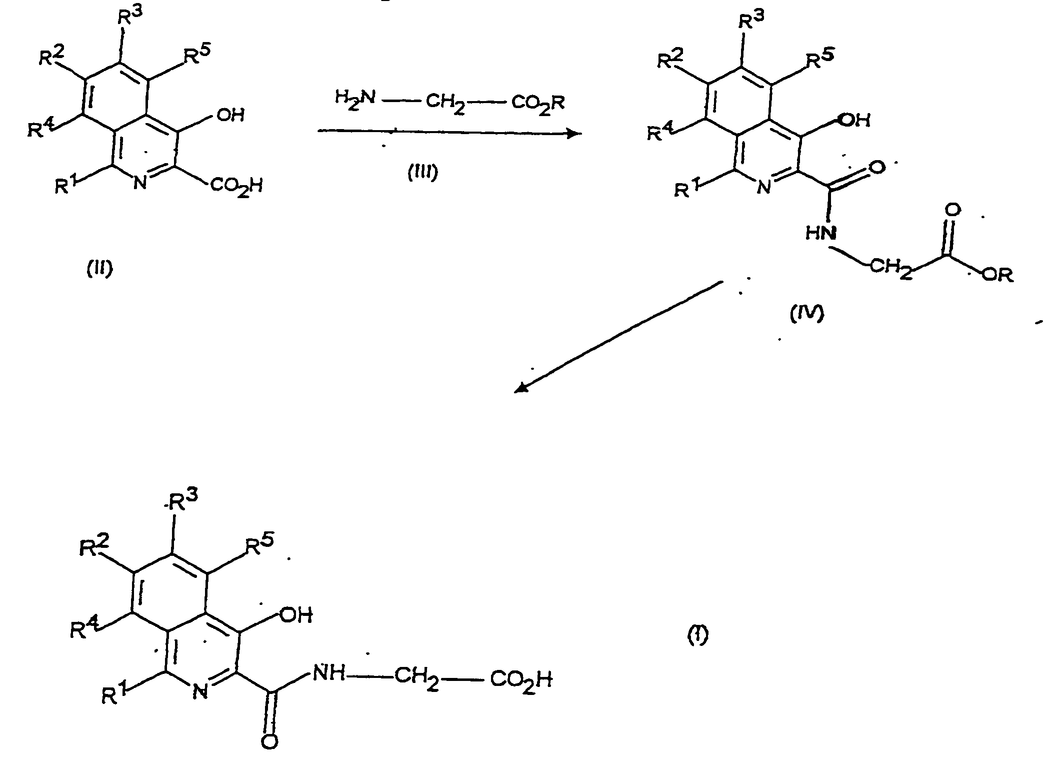

13. Verwendung einer Verbindung gemäß den Ansprüchen 1-11 zur Herstellung eines Arzneimittels zur Inhibierung der Kollagenbiosynthese.

14. Verwendung einer Verbindung gemäß den Ansprüchen 1-11 zur Herstellung eines Arzneimittels zur Hemmung der Prolyl-4-hydroxylase.

15. Verwendung einer Verbindung gemäß den Ansprüchen 1-11 zur Herstellung eines Arzneimittels zur Anwendung als Fibrosuppressiva.

16. Verwendung einer Verbindung gemäß den Ansprüchen 1-11 zur Herstellung eines Arzneimittels gegen fibrotische Erkrankungen.

17. Verwednung einer Verbindung gemäß den Ansprüchen 1-11 zur Herstellung eines Arzneimittels gegen fibrotische Erkrankungen der Leber, der Niere, der Lunge oder der Haut.

18. Arzneimittel enhaltend eine Verbindung gemäß den Ansprüchen 1 bis 11.

## Claims

1. Compounds of the formula I 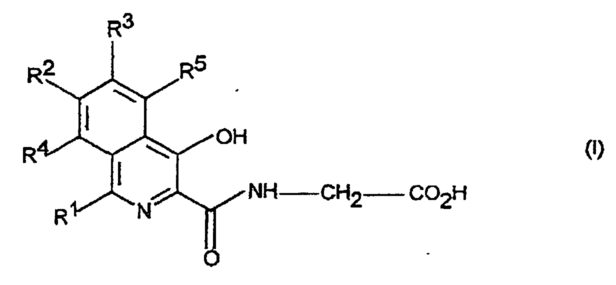 in which
R¹ is chlorine,
R² is hydrogen, (C₁-C₈)-alkyl, (C₁-C₈)-alkoxy, chlorine, trifluoromethyl, hydroxyl or benzyloxy, which is optionally substituted by substituents from the group consisting of (C₁-C₅)-alkyl and (C₁-C₅)-alkoxy, or fluoroalkoxy of the formula
O-[CH₂]ₓ-C_{f}H_{(2f+1-g)}F_{g}
where
x = 0 or 1,
f = 1 - 5 and
g = 1 to (2f+1),
R³ is hydrogen, (C₁-C₈)-alkyl, (C₁-C₈)-alkoxy, fluorine, chlorine, cyano, trifluoromethyl, hydroxyl, or benzyloxy which is optionally substituted by substituents from the group consisting of (C₁-C₆)-alkyl and (C₁-C₅)-alkoxy, or fluoroalkoxy of the formula
O-[CH₂]ₓ-C_{f}H_{(2f+1-g)}F_{g}
in which x, f and g are as defined above,
R⁴ and R⁵ are hydrogen, (C₁-C₅)-alkyl, fluorine, chlorine, bromine, trifluoromethyl, cyano, (C₁-C₅)-alkoxy, or fluoroalkoxy of the formula
O-[CH₂]ₓ-C_{f}H_{(2f+1-g)}F_{g}
in which x, f and g are as defined above,
including the physiologically active salts.

2. Compounds of the formula I according to Claim 1, in which
R¹ is chlorine,
R² is hydrogen, (C₁-C₈)-alkyl, (C₁-C₈)-alkoxy, chlorine, trifluoromethyl, hydroxyl, benzyloxy, or fluoroalkoxy of the formula
O-[CH₂]ₓ-C_{f}H_{(2f+1-g)}F_{g}
where
x = 0 or 1,
f = 1 -5 and
g = 1 to (2f+1),
R³ is hydrogen, (C₁-C₈)-alkyl, (C₁-C₈)-alkoxy, fluorine, chlorine, cyano, trifluoromethyl, hydroxyl, benzyloxy, or fluoroalkoxy of the formula
O-[CH₂]ₓ-C_{f}H_{(2f+1-g)}F_{g}
in which x, f and g are as defined above,
R⁴ is hydrogen, (C₁-C₈)-alkoxy, fluorine, chlorine, trifluoromethyl, cyano, or fluoroalkoxy of the formula
O-[CH₂]ₓ-C_{f}H_{(2f+1-g)}F_{g}
in which x, f and g are as defined above, and
R⁵ is hydrogen.

3. Compounds of the formula I according to Claim 1 or 2, in which
R¹ is chlorine,
R² is hydrogen, (C₁-C₅)-alkoxy, chlorine, benzylaxy, fluoroalkoxy where x=0, f=1,
R³ is hydrogen, (C₁-C₅)-alkoxy, chlorine or benzyloxy,
R⁴ is hydrogen, chlorine or methoxy,
R⁵ is hydrogen.

4. Compounds of the formula I according to Claim 1 or 2, in which
R¹ is chlorine,
R² is hydrogen, (C₁-C₈)-alkoxy, chlorine, hydroxyl or benzyloxy,
R³ is hydrogen, (C₁-C₈)-alkoxy, fluorine, chlorine, hydroxyl or benzyloxy,
R⁴ is hydrogen or chlorine,
R⁵ is hydrogen.

5. Compounds of the formula I according to Claim 1 or 2, in which
R¹ is chlorine,
R² is (C₁-C₈)-alkoxy, chlorine, or benzyloxy, and
R³, R⁴ and R⁵ are hydrogen.

6. Compounds of the formula I according to Claims 1 or 2, in which
R¹ is chlorine,
R² is hydrogen or chlorine,
R³ is (C₁-C₈)-alkoxy, chlorine or benzyloxy, and
R⁴ and R⁵ are hydrogen.

7. Compounds of the formula I according to Claim 1 or 2, in which
R¹ is chlorine,
R² and R³ are hydrogen or chlorine,
R⁴ is (C₁-C₆)-alkoxy, chlorine, or benzyloxy, and
R⁵ is hydrogen.

8. Compounds of the formula I according to Claim 1 or 2, in which
R¹ is chlorine,
R² is (C₁-C₆)-alkoxy or benzyloxy,
R³, R⁴ and R⁵ are hydrogen.

9. Compounds of the formula I according to Claim 1 or 2, in which
R¹ is chlorine,
R² is hydrogen,
R³ is (C₁-C₆)-alkoxy or benzyloxy, and
R⁴ and R⁵ are hydrogen.

10. Compounds of the formula I according to Claim 1 or 2, in which
R¹ is chlorine,
R² is (C₁-C₆)-alkoxy, and
R³, R⁴ and R⁵ are hydrogen.

11. N-((1-Chloro-4-hydroxyisoquinolin-3-yl)carbonyl)glycine, N-((1-chloro-4-hydroxy-7-((2-propyl)oxy)isoquinolin-3-yl)carbonyl)glycine.

12. Process for the preparation of compounds of the formula I according to Claims 1 to 11, **characterized in that** it comprises
1.i) reacting quinoline-2-carboxylic acids of the formula II where R is hydrogen, (C₁-C₈)-alkyl or benzyl with the amino esters of the formula III to give the amide esters of the formula IV, and
1.ii) releasing the compounds of the formula I from their esters of the formula IV.

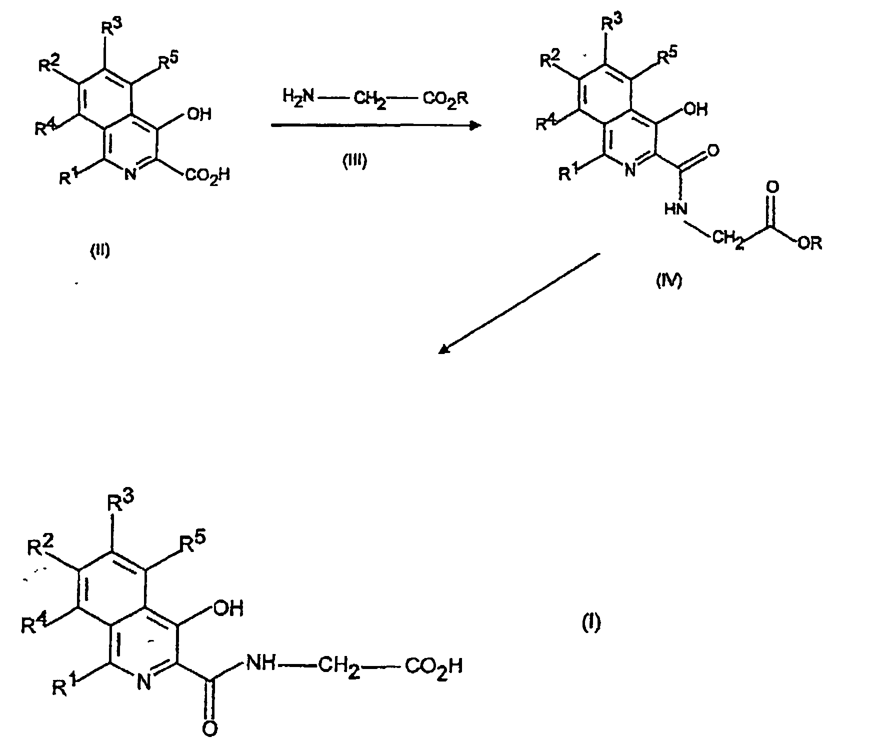

13. Use of a compound according to Claims 1-11 for the production of a pharmaceutical for the inhibition of collagen biosynthesis.

14. Use of a compound according to Claims 1-11 for the production of a pharmaceutical for the inhibition of prolyl-4-hydroxylase.

15. Use of a compound according to Claims 1-11 for the production of a pharmaceutical for use as a fibrosuppressant.

16. Use of a compound according to Claims 1-11 for the production of a pharmaceutical against fibrotic disorders.

17. Use of a compound according to Claims 1-11 for the production of a pharmaceutical against fibrotic disorders of the liver, the kidney, the lung and the skin.

18. Pharmaceutical comprising a compound according to Claims 1 to 11.

## Revendications

1. Composés de formule I 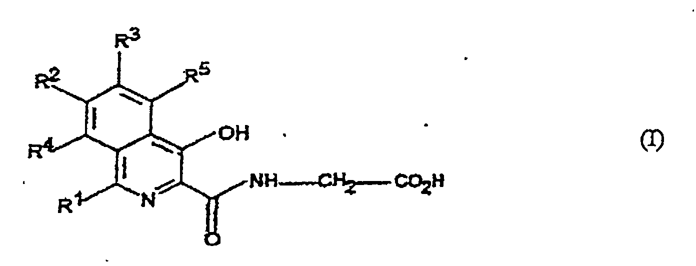 dans laquelle
R¹ représente le chlore
R² représente l'hydrogène, le radical alkyle en C₁-C₈, alcoxy en C₁-C₈, le chlore, le radical trifuorométhyle, hydroxy, ou benzyloxy, qui est substitué le cas échéant avec des substituants de la série radical alkyle en C₁-C₅, alcoxy en C₁-C₅, ou le radical fluoroalcoxy de formule
O-[CH₂]ₓ-C_{f}H_{(2f+1-g)}F_{g}
avec
x = 0 ou 1
f = 1 - 5 et
g = 1 à (2f+1),
R³ représente l'hydrogène, le radical alkyle en C₁-C₈, alcoxy en C₁-C₈, le fluor, le chlore, le radical cyano, trifuorométhyle, hydroxy, ou benzyloxy, qui est substitué le cas échéant avec des substituants de la série radical alkyle en C₁-C₅, alcoxy en C₁-C₅, ou le radical fluoroalcoxy de formule
O-[CH₂]ₓ-C_{f}H_{(2f+1-g)}F_{g}
dans laquelle x, f, et g sont définis comme ci-dessus,
R⁴ et R⁵ représentent l'hydrogène, le radical alkyle en C₁-C₅, le fluor, le chlore, le brome, le radical trifuorométhyle, cyano, alcoxy en C₁-C₅, ou fluoroalcoxy de formule
O-[CH₂]ₓ-C_{f}H_{(2f+1-g)}F_{g}
dans laquelle x, f, et g sont définis comme ci-dessus,
y compris les sels physiologiques actifs.

2. Composés de formule I selon la revendication 1, dans lesquels
R¹ est le chlore
R² est l'hydrogène, le radical alkyle en C₁-C₈, alcoxy en C₁-C₈, le chlore, le radical trifuorométhyle, hydroxy, benzyloxy ou fluoroalcoxy de formule
C-[CH₂]ₓ-C_{f}H_{(2f+1-g)}F_{g}
avec
x = 0 ou 1
f = 1 - 5 et
g = 1 à (2f+1),
R³ est l'hydrogène, le radical alkyle en C₁-C₈, alcoxy en C₁-C₈, le fluor, le chlore, le radical cyano, trifuorométhyle, hydroxy, benzyloxy ou fluoroalcoxy de formule
O-[CH₂]ₓ-C_{f}H_{(2f+1-g)}F_{g}
dans laquelle x, f, et g sont définis comme précédemment,
R⁴ est l'hydrogène, le radical alcoxy en C₁-C₈, le fluor, le chlore, le radical trifuorométhyle, cyano, ou fluoroalcoxy de formule
O-[CH₂]ₓ-C_{f}H_{(2f+1-g)}F_{g}
dans laquelle x, f, et g sont définis comme précédemment, et
R⁵ est l'hydrogène.

3. Composés de formule I selon la revendication 1 ou 2, dans lesquels
R¹ est le chlore
R² est l'hydrogène, le radical alcoxy en C₁-C₅, le chlore, le radical benzyloxy, fluoroalcoxy avec x = 0, f = 1,
R³ est l'hydrogène, le radical alcoxy en C₁-C₅, le chlore, le radical benzyloxy,
R⁴ est l'hydrogène, le chlore, le radical méthoxy,
R⁵ est l'hydrogène.

4. Composés de formule I selon la revendication 1 ou 2, dans lesquels
R¹ est le chlore
R² est l'hydrogène, le radical alcoxy en C₁-C₈, le chlore, le radical hydroxy, benzyloxy,
R³ est l'hydrogène, le radical alcoxy en C₁-C₈, le fluor, le chlore, le radical hydroxy, benzyloxy,
R⁴ est l'hydrogène, le chlore,
R⁵ est l'hydrogène.

5. Composés de formule I selon la revendication 1 ou 2, dans lesquels
R¹ est le chlore
R² est le radical alcoxy en C₁-C₈, le chlore, le radical benzyloxy et
R³, R⁴ et R⁵ sont l'hydrogène.

6. Composés de formule I selon la revendication 1 ou 2, dans lesquels
R¹ est le chlore
R² est l'hydrogène ou le chlore,
R³ est le radical alcoxy en C₁-C₈, le chlore, le radical benzyloxy, et
R⁴ et R⁵ sont l'hydrogène.

7. Composés de formule I selon la revendication 1 ou 2, dans lesquels
R¹ est le chlore
R² et R³ sont l'hydrogène ou le chlore,
R⁴ est le radical alcoxy en C₁-C₆, le chlore, le radical benzyloxy et
R⁵ est l'hydrogène.

8. Composés de formule I selon la revendication 1 ou 2, dans lesquels
R¹ est le chlore
R² est le radical alcoxy en C₁-C₆ ou le radical benzyloxy,
R³, R⁴, R⁵ sont l'hydrogène.

9. Composés de formule I selon la revendication 1 ou 2, dans lesquels
R¹ est le chlore
R² est l'hydrogène
R³ est un radical alcoxy en C₁-C₆ ou le radical benzyloxy et
R⁴ et R⁵ sont l'hydrogène.

10. Composés de formule I selon la revendication 1 ou 2, dans lesquels
R¹ est le chlore
R² est un radical alcoxy en C₁-C₆ et
R³, R⁴ et R⁵ sont l'hydrogène.

11. N-((1-chloro-4-hydroxyisoquinoléin-3-yl)carbonyl)glycine, N-((1-chloro-4-hydroxy-7-((2-propyl)oxy)isoquinoléin-3-yl)carbonyl)glycine.

12. Procédé de préparation de composés de formule générale I selon les revendications 1 à 11, **caractérisé en ce que**
1.i) les acides quinoléine-2-carboxylique de formule II sont transformés avec les esters d'amine de formule III en les esters d'amide de formule IV, R représentant l'hydrogène, le radical alkyle en C₁-C₈ ou benzyle, et
1.ii) les composés de formule I sont libérés de leurs esters de formule IV.

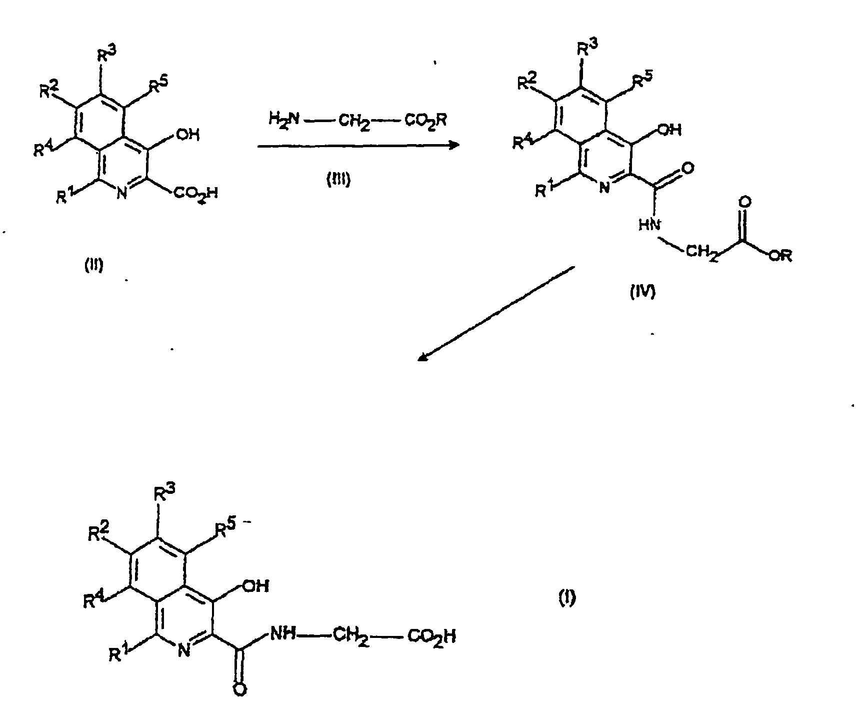

13. Utilisation d'un composé selon les revendications 1-11 pour la préparation d'un médicament pour inhiber la biosynthèse du collagène.

14. Utilisation d'un composé selon les revendications 1-11 pour la préparation d'un médicament pour bloquer la prolyl-4-hydroxylase.

15. Utilisation d'un composé selon les revendications 1-11 pour la préparation d'un médicament pour application en tant que fibrosuppresseur.

16. Utilisation d'un composé selon les revendications 1-11 pour la préparation d'un médicament contre les maladies fibrotiques.

17. Utilisation d'un composé selon les revendications 1-11 pour la préparation d'un médicament contre les maladies fibrotiques du foie, des reins, des poumons ou de la peau.

18. Médicament contenant un composé selon les revendications 1 à 11.
